# EUROPÄISCHE PATENTANMELDUNG

(11) **EP 2 042 180 A1**
(43) Veröffentlichungstag der Anmeldung: **01.04.2009**
(21) Anmeldenummer: 07117515.2
(22) Anmeldetag: 28.09.2007
(51) Int. Cl.: A61K 31/575, A61K 47/28, A61K 9/48, A61K 9/10

(54) **Phytosterol-haltige Präparate**

(71) Anmelder: Swiss Caps Rechte und Lizenzen AG, 9533 Kirchberg (CH)
(72) Erfinder: Brocker, Erich, 9533 Kirchberg (CH); Schneider, Marc, 9533 Kirchberg (CH)
(74) Vertreter: Welch, Andreas

(57) **Zusammenfassung**

Die vorliegende Erfindung betritt eine Weichkapsel mit einem Füllgut, **dadurch gekennzeichnet, dass** im Füllgut folgende Komponenten enthalten sind: Mindestens eine ernährungsphysiologisch oder pharmazeutische wirkende Substanz mit einem Cholestangerüst; ein zugesetztes Triglycerid, dessen Säurekomponenten aus Fettsäuren mit einer Kettenlänge von mehr als 8 Kohlenstoffatomen bestehen, in einer Menge von 10% bis 1000% der in der Gesamtmischung enthaltenen Menge an ernährungsphysiologisch oder pharmazeutische wirkender Substanz, ein Emulgator mit HLB >12 in einer Menge von 1-40% bezogen auf die Gesamtmischung, ein Co-Emulgator mit HLB<11 in einer Menge von 1-80% bezogen auf die Gesamtmischung, wobei das Füllgut selbstemulgierend und im Wesentlichen wasserfrei ist.

## Beschreibung

Die vorliegende Erfindung betrifft orale Darreichungsformen, insbesondere Weichkapseln enthaltend mindestens ein pharmazeutisch oder erwährungsphysiologisch wirksames Phytosterol, Phytostanol oder Phytoecdisteroid, insbesondere in einer besonders bioverfügbaren Zubereitung.

Phytosterole oder Phytostanole sind Gruppen von sekundären Pflanzeninhaltsstoffen mit Cholestan-Gerüst (I) die natürlicherweise in Pflanzen (und Pilzen) vorkommen und biosynthetisch über den Mevalonsäure-Isopren-Weg erschlossen werden. Sie können beispielsweise als Glycoside oder Ester vorliegen. Die bekanntesten Phytosterole oder Phytostanole wie Stigmasterol oder Campestrol weisen ein Cholesterol-Gerüst (II) auf:

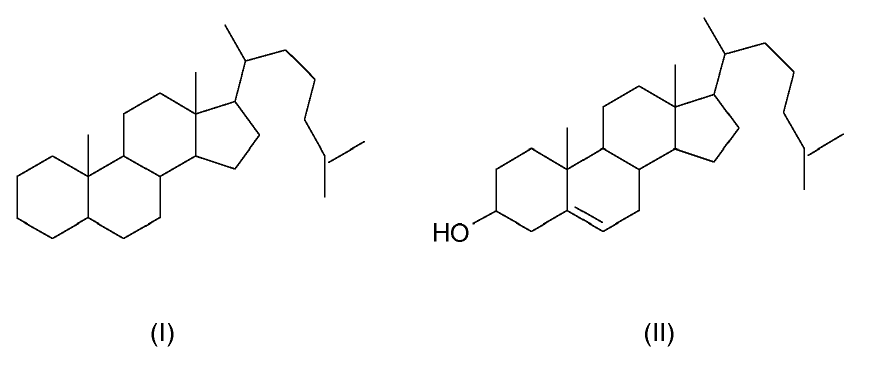

Im Folgenden werden die Substanzen mit Cholestanol-Gerüst (Delta-5 oder Delta-7) als PS abgekürzt bezeichnet.

Ähnliche Substanzen finden sich als sogenannte Phytoecdisteroide (Cholest-7-en-6-on Gerüst). Als wirksame Inhaltstoffe von bekannten Pflanzendrogen (wie z.B. Kürbis, Sabal serrulatum) wurden ebenfalls Sterole bzw. Sterolglycoside mit Gerüst (II) identifiziert.

Die Biosynthese führt zunächst zu Sesquiterpenen und Triterpenen. Kleinere Mengen der Triterpene werden zu Phytosterolen und zu Spuren von Phytoecdysteroiden direkt im Cytosol der Zelle verstoffwechselt. Es wird vermutet, dass die Phytosterole und Ecdysteroide in der Pflanze massgeblich an der Abwehr von Frassschädlingen beteiligt sind. Als relativ lipophile Substanzen werden sie vom Menschen hauptsächlich in der Fettfraktion der Lebensmittel aufgenommen. Isoliert sind es in der Regel weisse Pulver mit einem milden charakteristischen Geruch.

Zur Zeit stehen vor allem die in grösseren Mengen vorkommenden Phytosterole wie Beta-Sitosterol, Campestrol, Stigmasterol, Brassicasterol, Campestanol oder Sitostanol als Nahrungsergänzungsmittel (nutritional supplement) oder als wertgebender Nahrungsbestandteil im Interesse. Diese werden meist in roher Form (Phytosterolester-Komplex) oder teilweise fraktionierter Form mit einzeln angereicherten Phytosterolen aus Sojabohnen, Pinien oder andern fettreichen Pflanzenteilen (Nüssen, Getreide, Gemüse) extrahiert. Ein Komplex aus Beta-Sitosterol (<80%) Beta-Sitostanol (<15%), Campestrol (<40%), Campestanol (<5%) Stigmasterol (<30%) Brassicasterol (<3%) wird unter dem Namen Vegapur FTE (freie Sterole, mp 135-14°C) oder Vegapur 95E (Phytosterol-fettsäureester, Smp. 50°C) vermarktet.

Den PS werden pharmakologische Wirkungen wie anti-inflammatorische, anticancerogene, anti-artherogene oder anti-oxidative Aktivität zugeschrieben. Ein Bezug der Aktivität der PS zu der Aktivität von Hormonen ist wahrscheinlich, da die PS strukturelle biosynthetische Vorläufer von Hormonen sind.

Auch einige bekannte Phytotherapeutika basieren vermutlich auf der Wirkung von Phytosterolen (Saw palemetto, Pygeum africanum, Extr. Cucurbitae, Extr. Panicum miliaceum in den öligen (Mono-Di-Triglycerid) Extrakten.

Für die Zukunft ist zu erwarten, dass die bereits in der Nahrung in grösseren Mengen vorkommenden, bzw. der Nahrung zugesetzten Bulk-Phytosterole eher indifferente Wirkung entfalten, während weniger häufig vorkommende, noch nicht für den Einsatz beim Menschen "entdeckte" Phytosterole eine selektivere Rolle spielen werden. Die vorliegende Erfindung zielt deshalb darauf ab, vor allem hoch aufkonzentrierte, hochgereinigte und selektiv wirksame Phytosterole, Phytostanole und Phytoecdisteroide als Nahrungsergänzungsmittel und Arzneimittel zu formulieren.

Unter dem Begriff "Extrakte" sind alle Auszüge aus Pflanzenmaterial (selektiver oder mehrerer Pflanzenorgane) unter teilweiser Weglassung von primären Pflanzeninhaltsstoffen wie Cellulose, Saccharide, Proteine, Triglyceride zu verstehen. Der Zweck dieser Auszüge besteht in der Anreicherung bestimmter sekundärer Pflanzeninhaltsstoffe, insbesondere solchen mit erwünschten pharmakologischen Eigenschaften. Insbesondere können lipophile Extrakte durch Ausziehen der natürlich vorhandenen Öle/Fette oder fettlöslichen Fraktionen im Pflanzengewebe erhalten werden. Dabei können die natürlich vorhandenen Triglyceride/Wachse /Kohlenwasserstoffe auch die Rolle des Extraktions-Lösemittels übernehmen. Diese können auf Grund mangelnder Flüchtigkeit nicht abgedampft werden, sondern sind zur weiteren Anreicherung sekundärer Pflanzeninhaltsstoffe erneut mit einem Lösemittel, Gegenstromextraktion, überkritischer Extraktion, Chromatographischen Verfahren, (Wasserdampf- oder Vakuum) Destillation oder andern Trennverfahren weiter zu behandeln.

Lipophile Extrakte sind auch direkt erhältlich durch Pressen der Ölfraktion beziehungsweise durch Auszug mit einem lipophilen niedermolekularen Lösemittel wie hochprozentigem Ethanol, Aceton, Ethylacetat, Hexan, Chloroform oder überkritischem Kohlendioxid, welches abgedampft werden kann. In der Praxis werden oft Mischungen von Wasser mit Ethanol verwendet wodurch eine selektive Steuerung der ausgezogenen Inhaltstoffe ermöglicht wird. 40-80% Ethanol gilt als "mittelpolares" Auszugsmittel.

Dem Fachmann ist die Zerkleinerung der Pflanzenmaterials, der Aufschluss der Pflanzenzellen durch Mahlen, Erhitzen, Ultraschallbehandlung, sowie das Extrahieren, Anreichern durch Verdampfen des Lösemittels und das Reinigen hinreichend bekannt.

Eine Fraktionierung in reine Phytosterole kann nach Verseifung der Glycoside durch Flüssigchromatographie oder andere geeignete Verfahren erfolgen.

Die Bezeichnung lipophil kann für Substanzen verwendet werden, die in Octanol/Wasser-oder Octanol/Natriumchlorid-Lösung einen Verteilungskoeffizienten (log p) >1 haben.

Bedingt durch den lipophilen Charakter kommen Phytosterole verschwägert mit der Fettfraktion vor, wobei diese Fettfraktion entweder a) ein Rest aus der Extraktion (Quelle) ist, oder b) bei der Zubereitung der Darreichungsform (Zubereitung) zugegeben wurde oder c) zusammen mit Fetten aus der Nahrung im Magendarmtrakt (FOOD Interaktion) aufgenommen wurde. Eine gewisse Menge Fett erhöht die Wahrscheinlichkeit der Aufnahme von Phytosterolen, da der menschliche Körper auf die Verarbeitung von Triglyceriden eingerichtet ist. Cholesterol und wahrscheinlich auch die PS spielen eine Rolle bei der Emulgierung und Resorption von Mono- und Diglyceriden aus dem Darmtrakt. Eine kompetitive Hemmung der Nahrungs-Cholesterin-Aufnahme durch PS wird vermutet. Die Zugabe von Fett zu Phytosterolen oder die Aufnahme von Fetten, die Phytosterole enthalten, oder auch die Anreicherung von Nahrungsfeten mit Phytosterolen (Margarine) mag sinnvoll erscheinen, solange es sich um einen physikalischen Effekt bei der Aufnahme von Cholesterol handelt, ist aber keine sinnvolle Massnahme, sobald es um die gerichtete Ausnützung der ernährungsphysiologischen oder pharmakologischen Wirkung der Phytosterol und deshalb um eine gezielte Aufnahme selektiver PS in den Körper (Bioverfügbarkeit) geht. Zu diesem Zweck ist das wirksame PS zu resorbieren und soll in vivo die Wirkung entfalten.

Für die erfindungsgemäss bevorzugten Zielindikationen "Blutlipidsenkung" "Cholesterolsenkung" ist es weder wissenschaftlich sinnvoll begründbar, dem Konsumenten zuzumuten, besonders viel Fett angereichert mit PS zu sich zu nehmen. Ausserdem ist es erfahrungsgemäss für Konsumenten schwierig, die Nahrung in "gute Fette" und "böse Fette" zu trennen. Eine gezielte Dosis Phytosterol setzt eine kontrollierte Dosis (Menge) und die richtige chemische Konstitution (Monosubstanz, gereinigter Extrakt) und wirksame Aufnahme voraus. Dies ist nur möglich mit der richtigen Darreichungsform und Zubereitung. Unter oralen Darreichungsformen sind einzeldosierte Zubereitungen wie Tabletten, überzogene Tabletten, Dragees, Hartkapseln, Lozenges, Pastillen, Gums, Kaugummis, Filme, Brausetableten, Brausegranulate, Sherbets usw zu verstehen.

Ihnen gemeinsam ist, dass der interessierende pharmazeutisch oder ernährungsphysiologische Stoff einzeln oder kombiniert mit anderen Stoffen gezielt in eine Matrix eingebracht wird. Die Darreichungsformen werden geschluckt, in der Mundhöhle durch Speichel gelöst oder vorgängig in Flüssigkeit gelöst und dann geschluckt. Durch die Auswahl der Matrix bzw. durch die Steuerung der Eigenschaften der Matrix kann die Umlösung oder Emulgierung (Freisetzung) des Wirkprinzips in Speichel oder Magen- und Darmsaftsaft, und indirekt so auch die Bioverfügbarkeit gesteuert werden. Es gilt allgemein als anerkannt, dass je wasserlöslicher ein Stoff ist, bzw. je kleiner und polarer das Molekül ist, desto höher (und weniger beeinflusst durch andere Faktoren) die Bioverfügbarkeit ist.

Eine Erhöhung der Bioverfügbarkeit kann durch die Auswahl zugelassener Hilfsstoffe und die Zubereitung, sowohl für Nahrungsergänzung als auch Arzneiprodukte realisiert werden.

Zu einer erhöhten Bioverfügbarkeit tragen bei:
- Aktiver Stoff in Lösung
- Lösung bzw. flüssige Matrix, welche mit Körpersäften misch- oder emulgierbar ist
- Feinste /feinstverteilte Wirkstoffpartikel
- Erhöhung der Membranbindung der Träger-/ Wirkstoffpartikel
- Erhöhung der Membrangängigkeit

Kapseln sind etablierte Darreichungsformen für Arzneimittel und Nahrungsergänzungsmittel. Sie sind als Kern-Hülle-Struktur ausgebildet, d.h. ein Inhaltsstoff beliebiger Konsistenz (der Kern, auch als Füllgut bezeichnet) ist von einer Hülle aus geeignetem Hüllenmaterial umgeben. Man unterscheidet hierbei insbesondere Hartkapseln und Weichkapseln.

Kapseln und Verfahren zu ihrer Herstellung sind hinlänglich bekannt (z.B. Stanley, J.P. "Soft gelatin capsules" in: Lachman et al. (Hrsg.) "The theory and practice of industrial pharmacy", Philadelphia, Lea & Febiger, 3rd edition (1986); Hofer et al. in: Fahrig, W.; Hofer, U. (Hrsg.) "Die Kapsel", Wiss. Verlagsgesellschaft mbH, Stuttgart; Paperback APV Band 7, 1. Aufl.,1981).

Bei Hartkapseln besteht das Hüllenmaterial aus einem eher dünnen Film (mit einer Dicke bis zu 200 µm), der aber dennoch formstabil ist. In der Regel ist die Hülle aus zwei sich ergänzenden und zusammenfügbaren Teilen ("body" und "cap") aufgebaut.

Die beiden Kapselhüllenteile werden in einem ersten Schritt aus wässrigen Lösungen von Polymeren geformt und getrocknet. In der Regel werden Polymere verwendet, die in Lösung einen Sol-Gel Übergangszustand aufweisen (Gelatine, HPMC (Hydroxypropylmethylcellulose) + Carrageenan, usw.). Bedingt durch die die Technologie der Herstellung können nur sehr eng begrenzte Zusammensetzungen von Polymerlösungen verwendet werden. Es können in der Regel keine Variationen der Zusammensetzung der Hülle vorgenommen werden, etwa um später Interaktionen mit dem Füllgut zu vermeiden, oder um die fertige Kapsel bei besonders niedrigen oder besonders hohen Feuchten stabil zu halten.

In einem zweiten Schritt erfolgt das Trennen von Ober- und Unterteil, das Einfüllen des Füllgutes in das Unterteil und das erneute zusammenstecken der beiden Hüllenteile. In der Kapsel bleibt immer ein Gas (Luft) im Innenraum der Kapsel. Bei flüssigen Füllgütern oder Füllgütern, die sich während der Lagerung verflüssigen können, muss die zwischen Hüllenober- und unterteil bestehende Lücke mit einem weiteren Film verschlossen (Banderollieren, oder Spray-Film Coating) oder mit polymerem "Leim" verklebt werden.

Als Hüllenmaterial werden üblicherweise Gelatine, Cellulosederivate, Gummen, PVA (Polyvinylalkohol), PVP (Polyvinylpyrrolidon) und andere synthetische und natürliche Polymere oder Mischungen von Polymeren mit andern Stoffen verwendet. Hartkapseln werden in der Regel mit pulverigen, oder partikulären (Pellets) Füllgütern befüllt, seltener (aber auch) mit flüssigen, pastösen Füllgütern. Um das teure zusätzliche Verschliessen oder Coating zu vermeiden, wurden insbesondere auch Füllgüter entwickelt, die in geschmolzener Form eingebracht werden, bei Lagerbedingungen erstarrt sind und bei der Lagerung nicht schmelzen und auslaufen können.

Durch Erstarrung verfestigte Schmelzen lipophiler Stoffe sowie hochviskose Suspensionen von wasserlöslichen Partikeln in lipophiler Matrix eignen sich zur Befüllung von Hartkapseln: Hartkapseln auf der Basis von Gelatine oder HPMC enthalten kaum Weichmacher und Wasser (aw= 0.5, ca. 8% Wasser für Gelatine und ca. 4% für HPMC als Grundmaterial). Demzufolge sind deren mechanischen Eigenschaften auch ziemlich tolerant gegen lipophile Stoffe und Temperaturen bis 80°C. Mit anderen Worten schmelzen oder verformen sich die Hüllen nicht bei Temperaturen von 25-60°C.

Die Befüllung von Hartkapseln mit wachsartigen Stoffen wie Fetten, Partialglyceriden, Fettsäureestern (Polyethylenglycol-Fettsäureester, Gelucire) und langkettigen Polyethylenglycolen (PEG) ist bekannt. Solche Formulierungen eignen sich vor allem für die Formulierung von retardierten, langsam freisetzenden pharmazeutischen Präparaten, oder zur Formulierung von schwer wasserlöslichen aktiven Stoffen in wasserfreien, aber wasserlöslichen Matrizes.

Bei Weichkapseln ist die Hülle in der Regel dicker (mit einer Dicke über 200 µm). Das Hüllenmaterial enthält einen Weichmacher. Bedingt durch das Herstellungsverfahren bestehen Weichkapseln in der Regel aus einteiligen Filmen, d.h. Hüllen die auch gegen nicht wässrige Flüssigkeiten dicht sind. Als Filmmaterial hat sich in der Vergangenheit vor allem Gelatine bewährt. Die Herstellung erfolgt mit Hilfe von folgenden Techniken: Tropfverfahren, Ringextrusionsverfahren, injection moulding (Spritzgussverfahren), Coinjection moulding-Verfahren oder Verschweissung zweier halbschaliger Filme (z.B. Colton-, Upjohn-, Accogel-, Norton- oder Rotary die-Prozess).

Um die notwendige Flexibilität der Filme bei der Vertropfung oder der Formung und Verschweissung sicherzustellen, bestehen die Filme aus wässrigen Lösungen enthaltend Polymer, Weichmacher und Zuschlagstoffe. Das überschüssige Wasser wird durch Trockenen der geformten und befüllten Kapsel bis zum dem für die Lagerung notwendigen Anteil entfernt.

Weichkapseln werden in der Regel mit flüssigen oder pastösen Füllgütern (Suspension) befüllt. Sie können im Prinzip auch - mit Hilfe einer speziellen Dosiervorrichtung - mit Partikeln (Granulat oder Pellets) suspendiert in flüssigen Phasen oder mit pulverigen oder partikulären (Pellets) Füllgütern (ohne flüssige Phase) befüllt werden. Bevorzugt, da kaum Wechselwirkungen zwischen hydrophiler Hülle und Füllgut eintreten, sind liphophile flüssige Füllstoffe allein oder als Trägermatrix für eine Suspension von kristallinen wasserlöslichen Stoffen.

Die Verfahrensparameter (Temperatur, Druck, Zeit) beim Einbringen von Füllgütern in Weichkapseln sind bedingt bzw. limitiert durch die Bedingungen, welche für die gleichzeitige Verformung und Versiegelung der zwei Hüllenteile angewandt werden müssen. So ist bei der traditionellen Weichgelatinekapselherstellung aus wässrigen Gelatinelösungen (nach dem Rotary Die Verfahren, vgl. Bauer. in: Fahrig, W.; Hofer, U. (Hrsg.) "Die Kapsel", Wiss. Verlagsgesellschaft mbH, Stuttgart; Paperback APV Band 7, 1983, S. 70) bedingt durch den Gel-Sol-Übergang der wässrigen Gelatinebänder (bei ca. 40-45°C) die bei der Kapselherstellung tolerierbare Temperatur auf ca. 35°C beschränkt (US-6,352,717, Sp. 1, Z. 60-67). Analog ist bei Verwendung von oder Stärke/Carrageen-Wasser-Lösungen, ebenfalls bedingt durch den Sol-Gel-Übergang (bei ca. 50-70°C), die bei der Befüllung des Füllgutes anwendbare Temperatur auf maximal ca. 50°C beschränkt. Ausserdem ist das in den Filmen enthaltene Wasser oft problematisch für die Füllgüter.

Für spezielle Zusammensetzungen kann es gelingen, die Füllgutschmelzen durch Scherviskosität am Erstarren zu hindern, oder eine kurzfristige Unterkühlung der Schmelze vor Verbringung in die Weichgelatinekapsel durch spezielle Abfüllvorrichtungen zu erreichen. Derartige Methoden sind in der US 6352717 offenbart.

Das Einbringen von geschmolzenen Füllgütern zwischen zwei einhüllende Bänder mittels Extrusion / Kalandrierung wurde in der EP-0 799 012 A1 für die Herstellung von Tabletten beschrieben. Diese Technik ist aber insbesondere bei der Kalandrierung von konzentrischen Strängen (Füllgut innen, von äusserer Hülle umgeben) nicht als die Herstellung kapselförmiger Körper zu bezeichne, da die Menge an Füllgut und die Filmdicke sich am Rand der Kalanderform auf Null verjüngt und daher keineswegs eine relativ gleichförmige Hüllendicke rund um den Formkörper zu erreichen ist. Die so entstehenden Gräte verunmöglichen das Einnehmen, und die Verdünnung der Hülle erschwert die Kontrolle der galenischen Eigenschaften (zum Beispiel bei magensaftresistenter Ausprägung der Hülle).

Tabletten sind Komprimate von festen, kristallinen oder amorphen Stoffen. Praktisch alle festen Materialien (lipophil, hydrophil, Polymere) können in Tabletten verarbeitet werden. Tabletten erhalten Festigkeit durch das ineinander Verschränken der Partikel und haben in der Regel eine hohe innere Oberfläche, ausser - was gerade bei lipophilen wachsartigen Stoffen der Fall ist - wenn die Materialien bei der Kompression sich verformen und zusammensintern.

Es gelingt durch die Wahl eines fett- oder wachshaltigen Stoffes und gleichzeitigem Vorliegen von amphoteren Substanzen oder Emulgatoren in der Matrix, auch lipophile Stoffe ausreichend bioverfügbar in einer Tablette (Granulat, überzogene Tablette, Dragee, Lozenge, befüllte Hartkapsel) zu formulieren. Dazu sind insbesondere feste, in pulverigen oder Granulat-Form existierende Emulgatoren und Wachse auszuwählen. Ebenso kann die Wahl von lipiden Stoffen für das Hot-melt-Granulierverfahren eine geeignete Aufbereitung der Matrix zusammen mit dem Wirkstoff für das Tablettierverfahren darstellen.

Eine Tablette mit mikronisierten PS ist in WO 2007/038596 beschrieben. Diese bettet die PS allerdings nicht in eine lipide Matrix ein und zeigt auch keine Daten zur Freisetzung oder Bioverfügbarkeit.

Es ist bekannt (Peter J.H. Jones et al., Journal of Lipid Research 44, 1713 (2003), dass PS ohne gleichzeitige Gabe von Fetten keine Cholesterinsenkung zeigen.

Es hat sich gezeigt, dass die Einbettung von PS in lipidhaltige Matrizes durchaus einfach ist, aber der Forderung nach erhöhter Bioverfügbarkeit nicht entspricht. Ein solches einfaches System ist z.B. in WO 01/32029 erwähnt. Die WO 00/04887 beschreibt die Zusammensetzung von PS und PS-Ester mit Omega-3-Fettsäuren, Fettsäureestern oder Triglyceriden. Auch die Verwendung von Selbst-emulgierenden Systemen, auch in Weichkapseln, wird erwähnt, ohne dass aber für den Fachmann eine ausreichend detaillierte, sinnvolle Zusammensetzung ersichtlich wäre.

WO 01/32029 erwähnt Microemulsionen von PS, gibt aber keine ausreichend detaillierte Mengenangaben.

In einer besonders geeigneten Form können die Ester der Phytosterole direkt die Eigenschaften der Formulierbarkeit in einer Darreichungsform haben und ausreichende Bioverfügbarkeit zeigen (Acuff et al. Lipids in Health and Disease 6(11 2007). Die Mischbarkeit mit oder Emulgierbarkeit in physiologischen Flüssigkeiten von pH 1 bis pH 7 ist hingegen nicht gegeben oder nur über das natürliche Fettverdauungssystem gewährleistet. Dies ist insbesondere bei Patienten mit Leber-Galle Problemen (Gallenblase weg) problematisch.

Die Weichkapsel bietet den Vorteil, lipophile Stoffe (Flüssig bis fest) zu verarbeiten. Vom Verbraucher ist sie besonders geschätzt, da die Form und die durch Speichel schnell quellbare Hülle besonders leicht einzunehmen ist. Die Hülle erlaubt überdies durch Farbgebung eindeutige gefällige Charakterisierung, sowie Licht- und Sauerstoff-Schutz.

Es war die Aufgabe der vorliegenden Erfindung, lipophile PS in Zubereitungen zur Nahrungsergänzung und Arzneimitteln in geeigneter hoch-bioverfügbarer Form als Weichkapsel bereitzustellen.

Dabei waren eine Reihe von Zielen zu erreichen und von Problemen zu lösen, um die Eignung der Systeme für den erfindungsgemässen Zweck sicherzustellen:
- Möglichst geringe Fettbelastung in einer Diät
- Molekulardisperses Angebot des Wirkstoffes (je feiner der Stoff verteilt, umso höher die potentielle Verfügbarkeit)
- Mischbarkeit mit oder Emulgierbarkeit in physiologischen Flüssigkeiten von pH 1 bis pH 7
- Hohe Membranbindung bzw. aktiver Transport durch die Darmzellen, und Aufnahme in die Lymphbahn-Blut in Form von Tröpfchen (Chylomikron)
- Sicherstellung des Transportes über Lymphe/Blut durch geeignete Transportform

Dabei war zu berücksichtigen, dass die einzelnen PS neben der gemeinsamen prinzipiellen Lipophilie durchaus Unterschiede bezüglich Schmelzpunkt, Löslichkeit und Molekulargewicht zeigen. Ausserdem war zu berücksichtigen, dass sie als Lipid- oder Phospholipid-ester, als Glycoside, und auch in freier Form vorkommen bzw. verabreicht werden können.

Gemäss der vorliegenden Erfindung wird die vorstehende Aufgabe gelöst durch eine Weichkapsel mit einem Füllgut, dadurch gekennzeichnet, dass im Füllgut folgende Komponenten enthalten sind:
a) mindestens eine ernährungsphysiologisch oder pharmazeutische wirkende Substanz mit einem Cholestangerüst
b) ein zugesetztes Triglycerid, dessen Säurekomponenten aus Fettsäuren mit einer Kettenlänge von mehr als 8 Kohlenstoffatomen bestehen, in einer Menge von 10% bis 1000% der in der Gesamtmischung enthaltenen Menge an Substanz a)
c) ein Emulgator mit HLB >12 in einer Menge von 1-40% bezogen auf die Gesamtmischung
d) ein Co-Emulgator mit HLB<11 in einer Menge von 1-80% bezogen auf die Gesamtmischung
wobei das Füllgut selbstemulgierend und im Wesentlichen wasserfrei ist.

Vorzugsweise verhält sich das Füllgut der erfindungsgemässen Weichkapsel in der Freisetzungsapparatur derart, dass es bei weniger als 150 rpm zu Partikeln mit max. Durchmesser von 100 µm selbstemulgiert. Erfindungsgemäss bevorzugt sollte die Selbstemulsifizierung des Füllgutes in der Freisetzungsapparatur nach 30 bis 60 Minuten abgeschlossen sein.

Erfindungsgemäss soll unter dem Begriff "selbstemulgierend" verstanden werden, dass die entsprechende Zusammensetzung bei Kontakt mit wässrigen Systemen eine Emulsion ausbildet, d.h. ohne wesentliche Einwirkung von Scherkräften eine möglichst hohe Dispersität erreicht wird.

Das Emulsions- und Dispergierverhalten lässt sich als Modell für den menschlichen Magen-Darmtrakt am besten in einer Freisetzungsapparatur studieren. Erfindungsgemäss wird unter der Freisetzungsapparatur die gemäss USP (United States Pharmacopoeia) definierte Freisetzungsapparatur nach der Paddelmethode verstanden. Die Darreichungsform wird hierbei in ca. 900 ml einer Mischung aus Wasser oder künstlichem Magen- oder Darmsaft gegeben, und die bei mässiger Bewegung (50-150 rpm des Paddels) selbst entstehenden Tröpfchengrössen werden gemessen.

Es konnte erfindungsgemäss gezeigt werden, dass ein Füllgut umfassend mindestens ein PS, eine lipophile Matrix, welche geeignet ist die Phytosterole zu lösen, sowie mindestens einen amphiphilen Stoff, welcher die Löslichkeit der Phytosterole nicht erniedrigt und mit Körpersäften wie Magen- und Darmsaft (Artificial Gastric Juice USP, Artificial Intestinal Fluid USP) ausreichend emulgiert, die vorstehende Aufgabe befriedigend erfüllt. Wasser in den Formulierungen sollte vermieden werden.

Eine Formulierung zur oralen Abgabe von PS, die ein hoch-disperses PS enthält (gelöst, molekulardispers), lagerstabil ist (ohne Rekristallisation der PS) und bei Kontakt mit Körperflüssigkeiten selbstemulgiert, ist vorteilhaft. Besonders vorteilhaft ist eine feste Prä-Emulsion.

Durch die Steuerung der Zusammensetzung lässt sich spontanes Emulgieren mit wässrigen Medien, zum Beispiel Magensaft oder Darmsaft, erreichen. Besonders geeignet sind dafür zunächst flüssige Prä-Emulsions-Zusammensetzungen. Die flüssige Zubereitung wird durch die Darmperistaltik unterstützt, zunächst schnell grob, dann zunehmend fein dispergiert.

Überraschend wurde gefunden, dass auch eine bei Raumtemperatur flüssige, vorzugsweise eine bei 37°C Körpertemperatur flüssige oder zumindest visköse Matrix, welche hohe Mengen an Phytosterol/Phytosterolester enthält, als selbstemulgierend formuliert werden kann, also bei Kontakt mit wässrigen Systemen möglichst hohe Dispersität ohne wesentliche Einwirkung von Scherkräften zeigt. Dazu werden idealerweise Triglyceride, mindestens ein Emulgator mit HLB>15 und mindestens ein Co-Emulgator mit HLB<11 verwendet.

Es hat sich gezeigt, dass PS als "cholesterinähnliches" Molekül bevorzugt im Verhältnis 1:10 bis 10:1 in Mono-Di-Triglyceriden gelöst werden kann, so dass durch Zusatz von oberflächenaktiven Stoffen aufgrund von Selbst-Emulgation ein effektives Glycerid-Tröpfchen - also eine liposomale Form - mit dem enthaltenen PS entsteht. Eine ausschliessliche Verwendung von oberflächenaktiven Stoffen, auch wenn diese PS lösen, führt nicht immer zu befriedigenden Resultaten bei Emulsionen/Kolloiden in wässrigen Systemen.

Andererseits ist die Löslichkeit gewisser PS in Triglyceriden (z.B. C8/C10 Triglyceriden) bei 20°C auf etwa 5-10% PS beschränkt, da sonst Rekristallisation erfolgt. Eine solche Kristallisation aus emulgierten Öltröpfchen ist auch bei Tröpfchengrössen von <1µm noch mikroskopisch feststellbar.

Als SMEDDS (Self-micro-emulsifying-drug-delivery system) gelten selbstemulgierende Systeme mit Tröpfchengrössen von weniger als 50 nm. Als SEDDS (Self-emulsifying-drug-delivery system) gelten selbstemulgierende Systeme mit Tröpfchengrössen von typischerweise 100-300 nm. Als SEDDS werden aber noch Formulierungen bezeichnet, die bis 50 µm grosse Tröpfchen liefern. Die relative Instabilität der in der Freisetzungsapparatur entstehenden Emulsionen ist kein Nachteil. Es hat sich im Gegenteil gezeigt, dass besonders stabile Emulsionströpfchen nicht besser bioverfügbar sind, da sie z.B. schlechteren Membrankontakt haben. Tröpfchengrössen von unter 10 µm während 30-120 min sind hinreichend stabil, um eine bessere Verfügbarkeit in der Darmpassage erwarten zu lassen. In der Freisetzungsapparatur emulgierende Systeme mit Tröpfchengrössen von mehr als 50 µm sind unstabil - sie rahmen auf - sind aber dennoch bevorzugt gegenüber der Darreichung von hochkonzentrierten, nicht selbst emulgierenden Phytosterolestern mit einem Schmelzpunkt von über 50°C, oder von Lösungen der Phytosterole in Lipiden, deren lipophile, flüssige Phasen sich nicht in Wasser dispergieren/emulgieren.

Einen wesentlichen Einfluss hat auch die Beachtung eines möglichst positiven Zeta-Potentials zur Erhöhung des Übertritts von der wässrigen Phase an die Oberfläche der Schleimhaut (Microvilli) des Gastrointestinaltraktes. Dies kann über die Polarität der ausgewählten Komponenten gesteuert werden.

Weiter wurde gefunden, dass auch feste disperse Matrizes in Weichkapseln formuliert werden können. Zwar ist es möglich, das System auf Komponenten aufzubauen, die zwischen 10-25°C flüssig sind. Überraschenderweise wird aber gerade die Neigung der PS zur Re-Kristallisation wirksam unterdrückt, wenn die Beweglichkeit der Moleküle durch den festen Zustand der Matrix beeinträchtigt ist. Durch die Einbettung der PS in eine feste Matrix kann eine wesentlich besser molekulardisperse Form mit wesentlich erhöhter Stabilität gefunden werden. Als disperse Matrix eignen sich Zusammensetzungen mit einem breiten Schmelzbereich, um Kristallisationen auch der einzelnen Hilfsstoff- Fraktionen(Matrix) zu verhindern.

Matrizes, die bei Lagertemperatur (10-30°C) fest sind und gegebenenfalls auch bei Körpertemperatur (37°C) noch fest sind (und damit nicht mechanisch dispergierbar sind), lassen sich als Schmelzen bei Temperaturen bis 60-100°C verarbeiten. Die Befüllung von Schmelzen über 35°C in Weichgelatinekapseln war bisher durch die Restriktion des Sol-Gel Überganges der Gelatine-Weichmacher-Wasser-Schmelze nicht möglich. Es wurde aber erfindungsgemäss gefunden, dass eine Weichkapsel, umfassend eine Hülle und ein Füllgut mit mindestens einem pharmakologisch oder physiologisch aktiven Stoff, mit derartigen Schmelzen als Füllgut befüllt werden kann, wenn die Hülle aus durch Schmelzextrusion thermoplastisch verarbeitbarem polymerem Material aufgebaut ist.

Das Hüllmaterial ist in diesem Fall aus durch Schmelzextrusion thermoplastisch verarbeitbarem polymerem Material aufgebaut. Unter Schmelzextrusion im Sinne der vorliegenden Erfindung ist eine Herstellung, Verformung und Verschweissung einer thermoplastischen Masse bei Drücken von mehr als 1 bar (vorzugsweise mehr als 10 bar, noch bevorzugter mehr als 100 bar) und Temperaturen von mehr als 60°C, bevorzugter mehr als 80°C, noch bevorzugter mehr als 100 °C zu verstehen.

Bei der Schmelzextrusion wird ein Band aus dem entsprechenden Materials geformt, indem das Material durch eine Breitschlitzdüse oder mit Hilfe von Blasverfahren in Filmform bei Viskositäten gebracht wird, die eine Verarbeitung unter Ausnutzung der Schwerkraft (Casting) nicht erlauben. Bei der Schmelzextrusion müssen daher notwendigerweise die Temperatur (um mindestens 20-50°C) sowie der Druck (auf mehr als 10 oder sogar mehr als 100 bar) gegenüber Umgebungsbedingungen erhöht werden. Gegebenenfalls kann dem thermoplastisch verarbeitbarem Material Weichmacher und/oder Wasser sowie gängige Additive wie beispielsweise Gleitmittel zugegeben werden.

Ein exemplarisches, erfindungsgemäss verwendbares Schmelzextrusionsverfahren ist in der EP-1 103 254 A1 beschrieben. Die Schmelzextrusion kann in einem Doppelschneckenextruder durchgeführt werden, wie er in der EP-1 103 254 A1 (Ausführungsbeispiel, Fig. 4) im Detail beschrieben ist. Auf die entsprechende Offenbarung der EP-1 103 254 A1 wird ausdrücklich Bezug genommen.

Erfindungsgemäss kann das durch Schmelzextrusion thermoplastisch verarbeitbare polymere Material ausgewählt werden aus der Gruppe bestehend aus Polyvinylpyrrolidon, Hydroxypropylcellulose, Hydroxypropylmethylcellulsose, Polyvinylalkohol, Polyvinylalkoholacetalen, Polethylenglycol-Polyvinylalkohol-Pfropfpolymer, Carbopol, Polymer aus Butylmethacrylat, 2-dimethylaminoethylmethacrylat und Methylmethacrylat im Verhältnis 1 : 2 : 1 , Polyethylacrylat, Methylmethacrylat, Polymethacrylsäure, Ethylacrylat, Polyethylacrylat, Methylmethacrylat, Trimethylammoniumethylmethacrylatchlorid, Hydroxypropylmethylcelluloseacetatsuccinat, Polyox, Stärke, Polymilchsäure, Polymilchsäurecoglycolid, Gelatine, Carrageenan, Casein, Gluten und deren Mischungen.

Beispiele für erfindungsgemäss geeignete Materialien sind die in der EP-1 103 254 A1 offenbarten stärkehaltigen Massen sowie die in der EP-1 586 436 A1 offenbarten Hüllmaterialien, wobei im letzteren Fall Hüllmaterialien auf der Basis von PVACL bevorzugt sind.

Die beispielsweise in der EP-1 103 254 A1 beschriebenen stärkehaltigen Massen sind homogenisierte, Stärke enthaltende Massen, enthaltend vorzugsweise mindestens 45 Gew.-% einer amorphen Stärke mit einem Amylopektingehalt von grösser oder gleich 50 Gew.-% bezogen auf das Gewicht der wasserfreien Stärke, Wasser, mindestens einen organischen Weichmacher in einem Anteil von mindestens 12 Gew.-% bezogen auf das Gewicht der wasserfreien Stärke, wobei der Staudinger-Index der homogenisierten Masse mindestens 40 ml/g, bevorzugt mindestens 50 ml/g und noch bevorzugter mindestens 60 ml/g beträgt. Erfindungsgemäss soll der Staudinger-Index in derselben Weise wie in der EP-1 103 254 A1 definiert verstanden werden. Auf die entsprechende Offenbarung der EP-1 103 254 A1 wird ausdrücklich Bezug genommen.

Alternative verwendbare Materialien sind in der EP-0 090 600 A1 beschrieben. Auf die entsprechende Offenbarung der EP-0 090 600 A1 wird ausdrücklich Bezug genommen.

Erfindungsgemäss können auch die in der EP-1 586 436 A1 beschriebenen thermoplastischen Polymer eingesetzt werden, die ausgewählt sind aus der Gruppe bestehend aus Polyvinylalkohol, Celluloseether, Polycaprolacton, Polyamiden, Polyacrylsäure, Polyvinylpyrrolidon, Polymilchsäure oder Polyvinylalkoholacetalen (PVACL), Derivaten oder Gemischen derselben. Auf die entsprechende Offenbarung der EP-1 586 436 A1 wird ausdrücklich Bezug genommen.

Erfindungsgemäss bevorzugt besteht die Hülle aus einem thermoplastischen, bei 37°C wasserlöslichen Polymer. Erfindungsgemäss weiterhin bevorzugt besteht die Hülle aus magensaft-resistenten oder pH-abhängig sich lösenden Polymeren.

Aus diesen Materialien können durch Schmelzextrusion Filme erzeugt werden, welche in üblichen Weichkapsel-Herstellungsverfahren wie beispielsweise dem Rotary-Die-Verfahren zu Weichkapseln verarbeitet werden können. Vorzugsweise werden die beiden Schritte (Schmelzextrusion und Verkapselung) in-line (d.h. kontinuierlich) durchgeführt.

Das Rotary-Die-Verfahren ist allgemein bekannt und beispielsweise in der EP-1 103 254 A1 beschrieben. Auf die entsprechende Offenbarung der EP-1 103 254 A1 wird ausdrücklich Bezug genommen.

Gemäss der vorliegenden Erfindung erfolgt das Einbringen des Füllgutes im Kapselformungsschritt (z.B. der Rotary-Die-Methode) im Fall eines bei Raumtemperatur festen Füllgutes ebenfalls bei den erhöhten Temperaturen, die im vorhergehenden Schritt der Schmelzextrusion eingesetzt werden. Das anschliessende Verschweissen der Filme zur Kapselbildung erfolgt ebenfalls bei den für die Schmelzextrusion angegebenen erhöhten Temperatur- und Druckbedingungen. Erfindungsgemäss ist in der Regel ein Druck anzulegen, welcher den Druck bei einem herkömmlichen Rotary-Die-Verfahren um den Faktor 10 übersteigt. Erfindungsgemäss bevorzugt sind hierbei Drücke, welche den Druck bei einem herkömmlichen Rotary-Die-Verfahren um den Faktor 15 bis 40 übersteigen, d.h. etwa im Druckbereich von 225 bis 600 bar liegen.

Erfindungsgemäss bevorzugt wird die Kapsel nach der Herstellung schockgekühlt d, um Füllgut glasig zu machen. Dies erfolgt vorzugsweise durch kalte Gase (Stickstoff, Luft, CO₂) oder durch ein durch kaltes Bad aus mit der Hülle verträglichen Flüssigkeiten.

Es sei erwähnt, dass im Fall von bei Raumtemperatur (25°C) flüssigen Füllgütern auch herkömmliche Weichgelatinekapseln verwendet werden können. In diesem Fall erfolgt die Verkapselung im Rotary-Die-Verfahren auf herkömmliche Weise unter herkömmlichen Bedingungen.

Der Übergang vom "festen" in den "plastischen" oder "flüssigen" Zustand kann bei niedermolekularen und kristallisierenden Substanzen mit dem sog. Schmelzpunkt beschrieben werden. Als Schmelztemperatur bezeichnet man die Temperatur, bei der ein Stoff schmilzt, das heißt vom festen in den flüssigen Aggregatzustand übergeht.

Für Gemenge und Gemische aller Arten eignet sich zur Beschreibung des Verhaltens am besten der sof. Tropfpunkt. Dies ist die Temperatur bei welcher das Füllgut unter Einfluss der Schwerkraft tropft. (z.B. Methode USP <741> class III)

Dieser Tropfpunkt ist unabhängig davon wann die "erste" oder "letzte" Komponente "flüssig" ist, sondern richtet sich nur nach dem "mittleren" Verhalten.

Komponenten für bevorzugte Matrixzusammensetzungen für flüssige, pastöse und feste selbst-emulgierende Zubereitungen für PS in Weichkapseln können ausgewählt werden aus folgenden Stoffen:
□ Triglyceride, z.B.:
   ○ Triglyceride mit mehrheitlich mehrfach ungesättigten Fettsäuren und mehrfach veresterten ungesättigten Fettsäuren wie Sojaöl, Olivenöl Sonnenblumenöl, Distelöl, Rapsöl, Leinöl, oder Erdnussöl.
   ○ Triglyceride mit mehrheitlich nur einfach ungesättigten Fettsäuren wie Kokosfett, Palmkernfett, Maiskeimöl, teilhydriertes Sojaöl, oder teilhydriertes Baumwollsamenöl.
   ○ Triglyceride mit ausschliesslich gesättigten Fettsäuren wie hydriertes Bauwoll-oder Sojafett oder Mittelkettige Triglyceride.
□ Emulgatoren mit einem HLB>15, z.B.:
   a) Polyoxyl hydriertes Castoröl wie Cremophor^{™} RH 40, Cremophor EL, Arlaton^{™} 650/ G Pharma
   b) Polyoxyethylensorbitanfettsäureester wie Tween^{™}, Crillet^{™}, Tagat^{™}, Polysorbat^{™}20/ 40/ 60/ 80/ 120.
   c) Polyoxyethylen fettsäureester wie Brij^{™} 35/ 56/ 58/ 76/ 78/ 97/ 99, Cremophor^{™} S9
   d) Polyethyleneglycolmonofettsäureester
   e) Polyoxyethylen-x-stearate wie Polyoxyethylen-12/ 20/ 30/ 40/ 50 -stearat, polyoxyl-32/ 150- disterarat.
   f) Polyglycerolfettsäureester wie Sakamoto SY-glister™ MCA-750/MO-750, Admul^{™}, Polyaldo^{™}, Cithrol^{™} 2621.
   g) Saccharosefettsäureester wie Ryoto Sugar ester^{™} S-1570, O (OWA)-1570, P-1570, S-1670P1670, M-1695, LWA-1570, Sucroester 15™,
   h) Polyoxyethylen-polyoxypropylene-Blockpolymer, wie Pluronic^{™} F108, F98, Poloxamer^{™} 184/ 185/ 188/ 407, Synperonic^{™}.
   i) Natriumlaurylsulfat
□ Co-Emulgatoren mit einem HLB<11, z.B.:
   o Freie Fettsäuren: u.a. Ölsäure. Linolensäure, Stearinsäure, Palmitoleinsäue, Palmitinsäure, Priolen^{™}, Pristeren^{™}.
   o Sehr langkettige Fettsäuren und Fettalkohole (Policosanols)
   ○ Monoglyceride wie Capmul^{™} MCM, Peceol^{™}, Maisine^{™}, Monomuls^{™}, Estol GMC/ GMCC/ GML/ GMM/ GMS, Imwitor 491/ 308, Tegomuls
   ○ Mono-und Di-Glyceride wie (E471), Geleol^{™}, Glycerolmono-dimyristat, Imwitor 31/ 742/ 900/ 960/ 988
   ○ Mono- oder Diglyceride von Speisefettsäuren, verestert mit Essigsäure (E472a), Milchsäure (E472b), Citronensäure (E472c) oder Weinsäure (E472d) oder deren Mischester (E472e,f): Imwitor372/ 375/ 377/ 380/ 390, Axol E61, Axol L62, Axol C63, Datamuls 43,37,4720,4820.
   ○ Phospholipide: Phosphatidylcholin, Phosphatidylserin, Phosphatidylethanolamin, Sphingoµmelin und deren Gemische mit Triglyceriden.
   ○ Vitamin E TPGS
   ○ Sorbitanester wie Sorbat 20, 40, 60 ,65 80, 83, 85
   ○ Polyalkylenglycolester wie z.B. Brij 30, 72
   Wirkstoffe: Phytosterole (as natürlich vorkommenden Eukaryonten und Prokaryonten) und Verbindungen davon (z.B. Verseifungs- oder Verseterungsprodukte), z.B.:

□ Freie Sterole, z.B. Delta-5-Sterole wie β-Sitosterol, Campestrol, Stigmasterol, Brassicasterol, Campestanol, β-Sitostanol; oder Delta-7-Sterole wie (22E)-(24S)-Ethyl-5α-cholesta-7,22,25-trien-3 β-ol, (24R)-Ethyl-5α-cholest-7-en-3β-ol
□ Sterol-β-D-Glvcoside wie β-Sitosterol-β-D-glucoside, β-Sitosterol-β-D-diglucoside
□ Sterol-Fettsäureester wie β-sitosterolpalmitat, β -Sitosterol-myristat, β -Sitosterol-laurat
□ Sterol-Glycosidfettsäureester wie β -Sitosterol-6-0-myristyl-β-D-glucosid, β-Sitosterol-6-0-lauryl-β-D-glucosid, β-Sitosterol-6-0-caprinoyl-β-D-glucosid
□ Phospholipide der Phytosterole
□ Extrakte aus folgenden Pflanzen (enthaltend fette Öle und PS):
   • Sojabohnen
   • Sägepalmenfrüchte (Sabal serrulata)
   • Südafrikanisches Sternengras (Hypoxis rooperi)
   • Brennesselwurzel (Urtica species),
   • Kürbissamen (Cucurbita pepo),
   • Zitterpappel (Populus remula),
   • Roggenpollen (Secale cereale),
   • afrikanische Pflaume (Pygeum africana)
   • Schisandra chinesis

Erfindungsgemäss sollten die einzelnen Komponenten im Füllgut im folgenden Verhältnis zueinander eingesetzt werden: Das zugesetzte Triglycerid sollte in einer Menge von 10% bis 1000% der in der Gesamtmischung enthaltenen Menge an ernährungsphysiologisch oder pharmazeutische wirkender Substanz vorhanden sein. Der Emulgator mit HLB >12 sollte in einer Menge von 1-40 Gew.-% bezogen auf die Gesamtmischung vorhanden sein, und der Co-Emulgator mit HLB<11 sollte in einer Menge von 1-80 Gew.-% bezogen auf die Gesamtmischung vorhanden sein.

Die erfindungsgemässen Weichkapseln können übliche Zusatzstoffe enthalten. Beispielsweise sind inerte kristalline Zuschlagstoffe wie Calciumtriphosphat geeignet, die Differenz der Wärmausdehnung der geschmolzenen Füllmatrix gegenüber der thermoplstischen Mischung der Hülle während des Erstarrungsvorgangs zu reduzieren, so dass es nicht zu einer partiellen Trennung der Kapselhülle von Inhalt kommt.

Die erfindungsgemässen Weichkapseln können als Nahrungsergänzungsmittel, Medizinprodukt oder Arzneimittel verwendet werden.

Die vorliegende Erfindung wird nachstehend anhand von nicht einschränkenden Beispielen und Zeichnungen näher erläutert. Es zeigen:
- Fig. 1: Die mit Dispersionstechnologie an einem Elektroakustik-Spektrometer ermittelte Tröpfchengrösse der Zubereitung einer Ausführugnsform gemäss Beispiel 1
- Fig. 2: Das Phasendiagramm einer SMEDDS gemäss Beispiel 2
- Fig. 3: Die Tröpfchengrösse einer SEDDS gemäss Beispiel 2
- Fig. 4:: Ein Phasendiagramm für eine Ausführungsform gemäss Beispiel 3
- Fig. 5:: Ein Phasendiagramm für eine Ausführungsform gemäss Beispiel 4

### Beispiel 1: Flüssige selbst-mikro-emulgierende Zubereitung von PS-haltigem Extrakt: (SMEDDS)

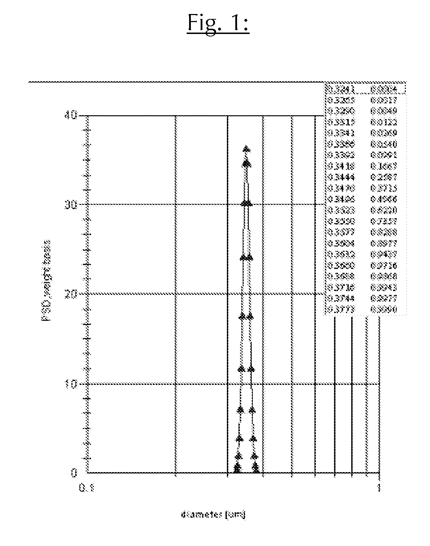

Phytosterolester aus Sabal serrulatum (0.2%w/w), gelöst in Triglyceriden aus der gleichen Pflanze (29.8%w/w (Ext. Sabal serrulatum oleosum), wurden mit Cremophor EL (35%w/w und Capmul MCM 35% w/w auf 50°C erwärmt und anschliessend unter Rühren abgekühlt. Die selbstemulgierenden Eigenschaften wurden getestet, indem ein Tropfen der jeweiligen Formulierung in 7 ml Wasser (entsprechend dem Verhältnis Kapselinhalt 350 mg in 900 ml Lösungsmedium) gebracht wurde, wobei die Reaktion nach leichtem Rühren beobachtet wurde. Es ergab sich eine praktisch transparente Flüssigkeit (bedingt durch die entstehende Tröpfchengrösse von durchschnittlich 350 nm). Die mit Dispersionstechnologie an einem Elektroakustik-Spektrometer DT 1200 ermittelte Tröpfchengrösse ist in Fig. 1 gezeigt.

### Beispiel 2: Selbstemulgierende Zubereitung von PS haltigem Extrakt (SEDDS)

Der Extrakt Sabal serrulatum oleosum wurde in verschiedensten Mengenverhältnissen analog zu Beispiel 1 aufgearbeitet und die entstehende flüssige Masse auf emulsifizierende Eigenschaft wie in Beispiel 1 geprüft. Je nach Tröpfchengrösse ergab sich eine "durchsichtige" SMEDDS bzw. eine trübe SEEDS-Emulsion.

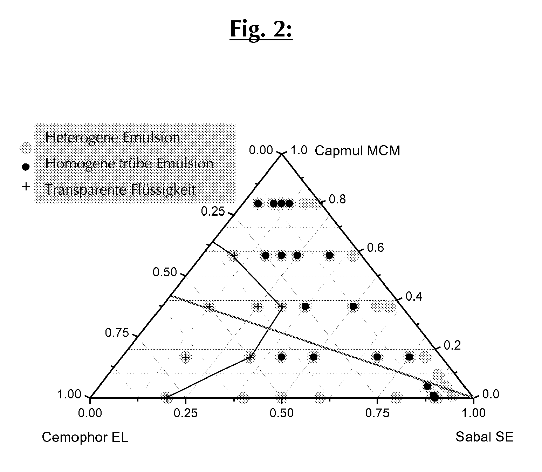

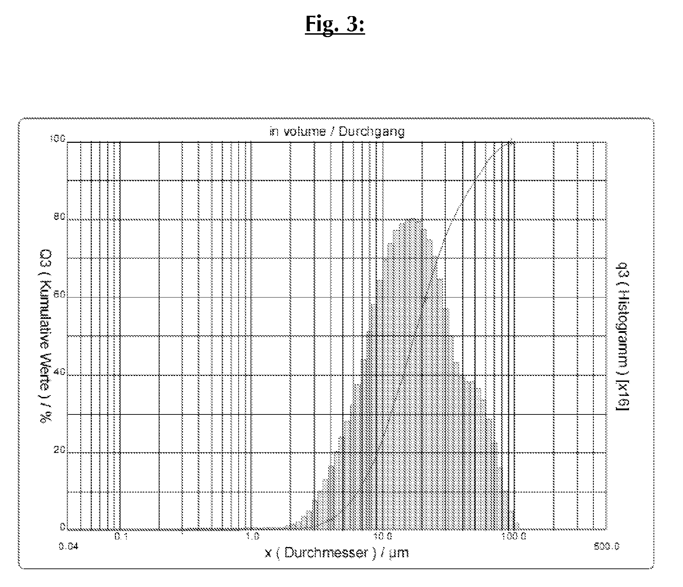

Die dabei beobachteten Verhältnisse wurden in ein Phasendiagramm eingezeichnet, welches für die SMEDDS mit Chremophor EL in Fig. 2 gezeigt ist. Die gemessene Tröpfchengrösse eines SEDDS mit Cremophor EL/ Capmul (Tröpfchenverteilung nach Selbstemulgation) ist in Fig. 3 gezeigt. Aus Fig. 2 ist zu erkennen, dass bei folgender Zusammensetzung eine SMEDDS oder eine SEDDS erhalten werden kann:

| | Extr. Sabal serrulatum | Cremophor EL | Capmul MCM |
|---|---|---|---|
| "SMEDDS" | 0-35% | 35-100% | 0-65% |
| "SEDDS" | 35-90% | 0-100% | 0-100% |

Es ist ersichtlich, dass ein selbstemulgierendes System mit SMEDDS-Charakter mit einem Anteil von maximal 35% Extrakt, und ein SEDDS-Kriterien genügendes System mit einem Anteil von bis zu 90% Extrakt realisiert werden kann.

### Beispiel 3:

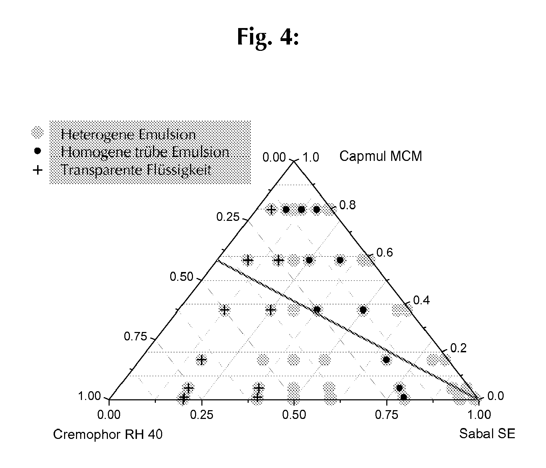

Analog zu Beispiel 2 wurden Systeme aus Sabal Extrakt mit Cremophor RH40 als Emulgator und Capmul MCM als Co-Tensid dargestellt. Die dabei beobachteten Verhältnisse wurden in ein Phasendiagramm eingezeichnet, welches in Fig. 4 gezeigt ist. Aus Fig. 4 ist zu erkennen, dass bei folgender Zusammensetzung eine SMEDDS oder eine SEDDS erhalten werden kann:

| | Sabal | Cremophor RH40 | Capmul MCM |
|---|---|---|---|
| "SMEDDS"* | 0-45% | 17-100% | 0-80% |
| "SEDDS"** | 10-90% | 0-100% | 0-100% |

### Beispiel 4:

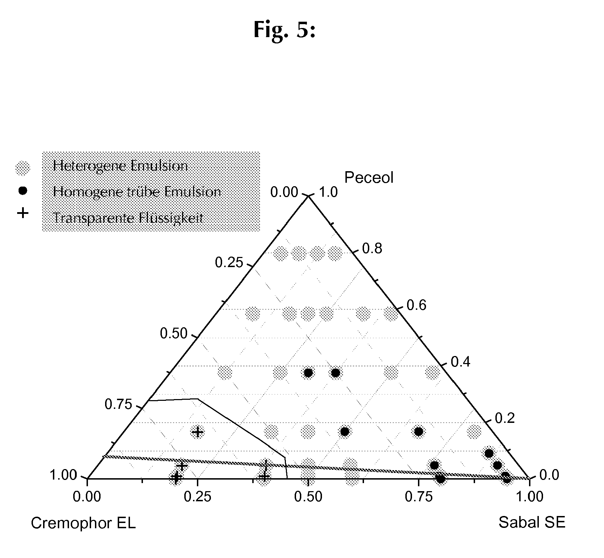

Analog den Beispielen 2 und 3 wurden Systeme aus Cremophor EL als Tensid und Peceol (Glyceyl-mono-oleat) als Co-Tensid dargestellt: Die dabei beobachteten Verhältnisse wurden in ein Phasendiagramm eingezeichnet, welches in Fig. 5 gezeigt ist. Aus Fig. 5 ist zu erkennen, dass bei folgender Zusammensetzung eine SMEDDS oder eine SEDDS erhalten werden kann:

| | Sabal | Cremophor EL | Peceol |
|---|---|---|---|
| "SMEDDS"* | 0-45% | 65-100% | 0-30% |
| "SEDDS"** | 30-98% | 1-35% | 0-45% |

### Beispiel 5:

Ein Phytosterolester Konzentrat mit 91 % Sterolestern und ca. 6% freien Sterolen VegaPur 95E (mp50°C) wurde in der folgenden Matrix bei 60° zu einer Lösung geschmolzen und unter Rühren abgekühlt:

| Funktion der Komponente | Stoff | Allgemeine Bezeichnung | mg |
|---|---|---|---|
| Aktiver Stoff | VegaPur 95E | Phytosterol Konz. 95% | 250 |
| Triglycerid | MCT | Mittelkettige Triglyceride | 25 |
| Emulgator | Decaglycerol-monolaurat | Syglister MC-750 | 25 |
| Co-Emulgator | Diacetylweinsäure-monoglycerid (E472e) | Imwitor 2020 | 6 |

Es ergab sich eine bei 25°C homogene Masse mit einem Tropfpunkt von 42 °C. Die Masse wurde bei 60°C in Weichkapseln Typ VegaGel^{®} (Stärkehülle hergestellt mit Schmelzextrusion) abgefüllt. Die Kapseln waren mechanisch stabil und dicht gesiegelt

Eine Prüfung auf Freisetzung ergab bei Kapselöffnung nach 20 min eine schwimmende Phase, die bei 150 rpm (Paddelrotation) innerhalb von 60 min in eine trübe, aber während 30 min stabile Emulsion überging.

### Beispiel 6:

200 mg Phytosterol-Konzentrat mit 99% Sterol VegaPur FTE (Smp 130°C) wurde unter Erhitzen und Rühren bis 130°C in 200 Polysorbat 80 (Tween 80) gelöst. Es wurden 20 mg Mittelkettige Triglyceride und 20 mg Glycerinmono-dioleat zugefügt. Bei Abkühlen auf 25°C ergab sich eine weisse halbfeste Masse, die bei Freisetzung in 200 mg Wasser fast klar in Lösung ging. Mikroskopisch konnten lamellenartige Strukturen und Tröpfchen von 0.1 bis 0.5 µm sowie nadelige Strukturen von 5 µm festgestellt werden.

Die Masse wurde bei 60°C in Weichkapseln Typ VegaGel^{®} (Stärkehülle hergestellt mit Schmelzextrusion) abgefüllt. Die Kapseln waren mechanisch stabil und dicht gesiegelt.

### Beispiel 7:

200 mg Phytosterol-Konzentrat mit 99% Sterol VegaPur FTE (Smp 130°C) wurden in eine Schmelze von 20 mg Hartfett (hydriertes Baumwollsamenöl, Smp 60°C), 200 mg Sucrosestearat (Smp. 49-56°C (Ryoto sugar ester S-1670 HLB16) und 20 mg Glycerinmonosterat (Monomuls 90-35 Cognis) eingemischt, in einem 2 Wellenextruder bei maximal 130°C geschmolzen und durch eine 0.6mm Lochdüse bei 80°C (rotierendes Schneidewerkezug) zu Pellets von ca. 0.5-1 mm Durchmesser geschnitten. Die Pellets wurden in der Freisetzungsapparatur bei 150 rpm geprüft: Innerhalb von 4 h bildete sich zuerst eine Emulsion von ca. 5 µm Partikeln. Anschliessend entstanden nadelige Mikrokristalle von ca 5 µm.

Die Pellets wurden ca. ca. 400 mg in Hartgelatinekapseln von Gr. 0 abgefüllt.

### Beispiel 8:

Die in Beispiel 6 erzeugte Schmelze wurde bei ca. 80°C über ein kleines beheiztes Zwischengefäss von ca. 1 liter Volumen drucklos direkt in die Dosierpumpe einer Rotary Die-Weichkapselmaschine zugeführt, die mit einem durch Schmelzextrusion erzeugten Thermoplasten als Band ausgerüstet wurde. Die Hülle der Kapsel bestand aus Polyvinylalkohol-stärkeacetal (PVACL). Die durchsichtige Kapsel mit weissem festen Inhalt war galenisch mehrere Monate stabil. Die Freisetzung - nach Öffnen der Kapsel nach 45 min - ergab das gleiche Emulgierverhalten wie unter Beispiel 6.

## Patentansprüche

1. Weichkapsel mit einem Füllgut, **dadurch gekennzeichnet, dass** im Füllgut folgende Komponenten enthalten sind:
a) mindestens eine ernährungsphysiologisch oder pharmazeutische wirkende Substanz mit einem Cholestangerüst
b) ein zugesetztes Triglycerid, dessen Säurekomponenten aus Fettsäuren mit einer Kettenlänge von mehr als 8 Kohlenstoffatomen bestehen, in einer Menge von 10% bis 1000% der in der Gesamtmischung enthaltenen Menge an Substanz a)
c) ein Emulgator mit HLB >12 in einer Menge von 1-40 Gew.-% bezogen auf die Gesamtmischung
d) ein Co-Emulgator mit HLB<11 in einer Menge von 1-80 Gew.-% bezogen auf die Gesamtmischung
wobei das Füllgut selbstemulgierend und im Wesentlichen wasserfrei ist.

2. Weichkapsel nach Anspruch 1, **dadurch gekennzeichnet, dass** die Substanz a) ein Delta-7-Sterol ist.

3. Weichkapsel nach Anspruch 1 oder 2, **dadurch gekennzeichnet, dass** die Substanz ein Sterol-Glycosid, Sterolester oder Glycosid-fettsäureester ist.

4. Weichkapseln nach Anspruch 1, **dadurch gekennzeichnet das** die Substanz ein Delta-5 Sterol (Cholesterol-Gerüst) aufweist.

5. Weichkapsel nach Anspruch 4, **dadurch gekennzeichnet, dass** die Substanz a) ein Sterol-Glycosid, Sterol-ester oder Sterol-Glycosid-fettsäureester ist.

6. Weichkapsel nach einem der Ansprüche 1 bis 5, **dadurch gekennzeichnet, dass** die Substanz a) in Eukaryonten oder Prokaryonten natürlich vorkommt, aus diesen gewonnen und gegebenenfalls verseift oder verestert wurde.

7. Weichkapsel nach einem der Ansprüche 1 bis 6, **dadurch gekennzeichnet dass** die Selbstemulsifizierung des Füllgutes in einer Freisetzugapparatur nach USP innerhalb von 30 min abgeschlossen ist.

8. Weichkapsel nach einem der Ansprüche 1 bis 7, **dadurch gekennzeichnet, dass** der Emulgator ausgewählt ist aus der Gruppe bestehend aus Polyoxyl hydriertes Castoröl, Polyoxyethylensorbitanfettsäureester, Polyoxyethylenfettsäureester, Polyethylenglycolmonofettsäureester, Polyoxyethylen-x-stearat, Polyglycerolfettsäureester, Saccharosefettsäureester, Polyoxyethylen-polyoxypropylen-Blockpolymer, und Natriumlaurylsulfat.

9. Weichkapsel nach einem der Ansprüche 1 bis 8, **dadurch gekennzeichnet, dass** der Co-Emulgator ausgewählt ist aus der Gruppe bestehend aus freien Fettsäuren, Monoglyceriden, Diglyceriden, Mono-diglyceridester , Mono-Diglyceride von veresterten Speisefettsäuren, Phospholipiden wie Phosphatidylcholin, Phosphatidylserin, Phosphatidylethanolamin, Sphingomyelin und deren Gemische mit Triglyceriden, Galactolipiden und Sorbitanestern.

10. Weichkapsel nach einem der Ansprüche 1 bis 9, **dadurch gekennzeichnet, dass** das Füllgut einen Tropfpunkt von mehr 25°C , bevorzugt von mehr als 37°C und noch bevorzugter von mehr als 55°C aufweist.

11. Weichkapsel nach einem der Ansprüche 1 bis 10, **dadurch gekennzeichnet, dass** die Hülle aus durch Schmelzextrusion thermoplastisch verarbeitbarem polymeren Material aufgebaut ist.

12. Weichkapsel nach Anspruch 11, **dadurch gekennzeichnet, dass** das durch Schmelzextrusion thermoplastisch verarbeitbare polymere Material ausgewählt ist aus der Gruppe bestehend aus Polyvinylpyrrolidon, Hydroxypropylcellulose, Hydroxypropylmethylcellulsose, Polyvinylalkohol, Polyvinylalkoholacetalen, Polethylenglycol-Polyvinylalkohol-Pfropfpolymer, Carbopol, Polymer aus Butylmethacrylat, 2-dimethylaminoethylmethacrylat und Methylmethacrylat im Verhältnis 1 : 2 : 1 , Polyethylacrylat, Methylmethacrylat, Polymethacrylsäure, Ethylacrylat, Polyethylacrylat, Methylmethacrylat, Trimethylammoniumethylmethacrylatchlorid, Hydroxypropylmethylcelluloseacetatsuccinat, Polyox, Stärke, Polymilchsäure, Polymilchsäure-coglycolid, Gelatine, Carrageenan, Casein, Gluten und deren Mischungen.

13. Weichkapsel nach Anspruch 11 oder 12, **dadurch gekennzeichnet, dass** das durch Schmelzextrusion thermoplastisch verarbeitbare polymere Material eine homogenisierte, Stärke enthaltende Masse ist, enthaltend vorzugsweise mindestens 45 Gew.-% einer amorphen Stärke mit einem Amylopektingehalt von grösser oder gleich 50 Gew.-% bezogen auf das Gewicht der wasserfreien Stärke, Wasser, mindestens einen organischen Weichmacher in einem Anteil von mindestens 12 Gew.-% bezogen auf das Gewicht der wasserfreien Stärke, wobei der Staudinger-Index der homogenisierten Masse mindestens 40 ml/g, bevorzugt mindestens 50 ml/g und noch bevorzugter mindestens 60 ml/g beträgt.

14. Weichkapsel nach Anspruch 11 oder 12, **dadurch gekennzeichnet, dass** das durch Schmelzextrusion thermoplastisch verarbeitbare polymere Material zusätzliche Stoffe enthält ausgewählt aus der Gruppe bestehend aus Weichmachern, Farbstoffen, UV-Stabilisatoren, Konservierungsstoffen, Antioxidantien, physikalisch und/oder chemisch modifizierten Biopolymeren, Zerfallsbeschleunigern, Formstabilisatoren, Gleit- und Entformmitteln und Trennmitteln.

15. Weichkapsel nach einem der Ansprüche 1 bis 9, **dadurch gekennzeichnet, dass** das Füllgut einen Tropfpunkt von weniger als 25°C aufweist.

16. Verfahren zur Herstellung von Weichkapseln gemäss einem der Ansprüche 1 bis 15, umfassend die Schritte
a) Bereitstellung eines Hüllmaterials
b) Bereitstellung eines Füllguts
c) Herstellung von Weichkapseln durch das Rotary-Die-Verfahren.

17. Verfahren nach Anspruch 16, **dadurch gekennzeichnet, dass** das Hüllmaterial wie in einem der Ansprüche 11 bis 14 definiert ist.

18. Verfahren nach Anspruch 17, **dadurch gekennzeichnet, dass** das Füllgut wie in einem der Ansprüche 1 bis 10 definiert ist und durch derartiges Aufschmelzen bereitgestellt wird, dass es pumpbar ist, als aufgeschmolzenes Füllgut in die Weichkapsel dosiert und die Kapsel anschliessend auf Umgebungstemperatur abgekühlt wird.

19. Verfahren nach Anspruch 16, **dadurch gekennzeichnet, dass** das Füllgut wie in Anspruch 15 definiert ist.

20. Verwendung einer Weichkapsel nach einem der Ansprüche 1 bis 15 als Nahrungsergänzungsmittel, Medizinprodukt oder Arzneimittel.
